# EUROPEAN PATENT APPLICATION

(11) **EP 4 144 883 A1**
(43) Date of publication of application: **08.03.2023**
(21) Application number: 21194805.4
(22) Date of filing: 03.09.2021
(51) Int. Cl.: C23C 14/48, C23C 14/12, G03F 7/00

(54) **LASER PRINTING AND PHOTOPOLYMERIZATION OF CELL-LADEN HYDROGELS**

(71) Applicant: PhosPrint P.C., 153 41 Agia Paraskevi, Athens (GR)
(72) Inventor: ZERGIOTI, Ioanna, 15669 Athens (GR); PAPAZOGLOU, Symeon, 114 73 Athens (GR); KLINAKIS, Apostolos, 15451 Athens (GR)
(74) Representative: Böhm, Brigitte

(57) **Abstract**

The present invention provides a laser-printing method for forming a hydrogel, especially a cell-laden hydrogel on a receiver substrate, and to an irradiation configuration comprising a donor substrate, a receiver substrate and a pulsed laser source for use in such method.

## Description

The present invention provides a laser-printing method for forming a hydrogel, especially a cell-laden hydrogel on a receiver substrate, and to an irradiation configuration comprising a donor substrate, a receiver substrate and a pulsed laser source for use in such method.

### Background

Hydrogels consist of 3D network structures from natural and/or synthetic polymers and have received great attention during the last decade since they mimic elements of native extracellular matrices (ECMs) and can promote many cellular functions, including cell adhesion, viability and proliferation. Natural polymers employed in bioprinting include collagen, Matrigel^{™}, alginate, hyaluronic acid, chitosan and fibrin hydrogels, while main synthetic hydrogels involve PHEMA (poly(hydroxyethyl methacrylate)), PEG (poly(ethylene glycol)), PEGDA (poly(ethylene glycol) diacrylate), platelet lysates modified by addition of methacryloyl groups (PLMA) and Gelatin methacryloyl (GeIMA) hydrogels.

Laser bioprinting has been reported by Gruene et al. (M. Gruene, C. Unger, L. Koch, A. Deiwick, B. Chichkov, Dispensing pico to nanolitre of a natural hydrogel by laser-assisted bioprinting, BioMedical Engineering OnLine, 10(19), (2011). doi:10.1186/1475-925X-10-19) for the printing of a hydrogel comprising alginate and ethylenediaminetetraacetic acid (EDTA) blood plasma and by Yusupov et al. (V. Yusupov, S. Churbanov, E. Churbanova, K. Bardakova, A. Antoshin, S. Evlashin, P. Timashev, N. Minaev, Laser-induced Forward Transfer Hydrogel Printing: A Defined Route for Highly Controlled Process, Int. J. Bioprint., 6(3): 271, (2020). DOI: 10.18063/ijb.v6i3.271) for the printing of hyaluronic acid sodium salt, methylcellulose, and sodium alginate hydrogels.

Laser bioprinting has been also employed for the printing of cell-laden hydrogels as a method to produce 3D structures with cells embedded in hydrogel matrices. Examples of cell-laden hydrogel bioprinting have been reported by Guillotin et al. (B. Guillotin, A. Souquet, S. Catros, M. Duocastella, B. Pippenger, S. Bellance, R. Bareille, M. Remy, L. Bordenave, J. Amedee, F. Guillemot, Laser assisted bioprinting of engineered tissue with high cell density and microscale organization, Biomaterials 31, 7250-7256 (2010). doi:10.1016/j.biomaterials.2010.05.055) where bioinks including rabbit carcinoma cell line B16 and human umbilical vein endothelial cell line Eahy926 have been supplemented with alginate and glycerol to produce free form tissue structures. In another study, Koch et al. *(*L. Koch, A. Deiwick, A. Franke, K. Schwanke, A. Haverich, R. Zweigerdt, B. Chichkov, Laser bioprinting of human induced pluripotent stem cells-the effect of printing and biomaterials on cell survival, pluripotency, and differentiation, Biofabrication, 10, 035005 (2018). doi.org/10.1088/1758-5090/aab981) employed laser bioprinting of bioinks containing human induced pluripotent stem cells and different hydrogels including collagen, alginate, hyaluronic acid, fibrinogen, fibrin, Geltrex^{™} and Matrigel^{™}.

Main methods for crosslinking hydrogels include chemical approaches using calcium chloride (CaCl₂) and photo-crosslinking using UV, visible or near infrared light. For example, Xiong et al. (R. Xiong, Z. Zhang, W. Chai, Y. Huang, D. B. Chrisey, Freeform drop-on-demand laser printing of 3D alginate and cellular constructs, Biofabrication 7 045011 (2015). doi:10.1088/1758-5090/7/4/045011) employed laser printing of alginate and cellular constructs while after printing the constructs where crosslinked in situ using CaCl₂. Compared to chemical crosslinking, photo-crosslinking approaches provide the ability to selectively crosslink the cellular constructs at precise locations and produce hydrogel structures with tunable properties. UV light photo-crosslinking (200-400 nm) has been demonstrated for example in the work of Bertassoni et al. (L. E. Bertassoni, J. C. Cardoso, V. Manoharan, A. L. Cristino, N. S. Bhise, W. A. Araujo, P. Zorlutuna, N. E. Vrana, A. M. Ghaemmaghami, M. R. Dokmeci, A. Khademhosseini, Direct-write Bioprinting of Cell-laden Methacrylated Gelatin Hydrogels, Biofabrication 6(2):024105, (2014). doi:10.1088/1758-5082/6/2/024105) and in WO 2013/158508 A1. Visible light photo-crosslinking (400-700 nm) has been reported in the work of Noshadi et al. (I. Noshadi, S. Hong, K. E. Sullivan, E. S. Sani, R. Portillo-Lara, A. Tamayol, S. R. Shin, A. E. Gao, W. L. Stoppel, L. D. Black, A. Khademhosseini, N. Annabi, In vitro and in vivo analysis of visible light crosslinkable gelatin methacryloyl (GelMA) hydrogels, Biomaterial Science 5, 2093 (2017). DOI: 10.1039/c7bm00110j*),* in the work of Wang et al. (Z. Wang, X. Jin, R. Dai, J. F. Holzman, K. Kim, An ultrafast hydrogel photocrosslinking method for direct laser bioprinting, RCS Advances, 6, 21099-21104, (2016). doi.org/10.1039/C5RA24910D) and in WO 2017/095240 A1, while more recently near infrared light at 980 nm (NIR) was employed for the photo-crosslinking of constructs comprising adipose-derived stem cells (ASCs) and articular chondrocytes in GelMA bioinks (Y. Chen, J. Zhang, X. Liu, S. Wang, J. Tao, Y. Huang, W. Wu, Y. Li, K. Zhou, X. Wei, S. Chen, X. Li, X. Xu, L. Cardon, Z. Qian, M. Gou, Noninvasive in vivo 3D bioprinting, Science Advances 6(23): eaba7406 (2020). DOI: 10.11261sciadvaba7406)*.*

UV photo-crosslinking is based on the use of photo-initiators such as Irgacure 2959. Although UV light has been widely employed for the photo-crosslinking of the aforementioned materials, it has been associated with safety concerns including DNA damage, accelerated aging of tissues and cancer. For visible light crosslinking, a number of photo-initiators have been used such as camphorquinone, fluorescein, riboflavin and rose Bengal, however they present limited photo-reactivity or exhibit cytotoxicity. A more recently synthesized photo-initiator is lithium phenyl-2,4,6-trimethylbenzoylphosphinate (LAP) that absorbs at 405 nm and provides good photo-reactivity and cytocompatibility. However, the synthesis route is complex and it is not yet commercially available.

From the above mentioned drawbacks of the previously described methods, it is evident that the subject of on-demand printing hydrogels on a desired surface, especially hydrogels which include live cells, has not yet been satisfactorily solved. It was accordingly an object of the present invention to provide an easily applicable and safe method which allows to transfer such hydrogels on any desired substrate using e.g. visible light without negatively affecting substances or cells included in the hydrogels.

### Summary of the Invention

In order to provide a solution to the above mentioned object, the present invention provides a method of forming a hydrogel on a receiver substrate, the method comprising
a) providing a hydrogel or a hydrogel-forming pre-polymer solution comprising visible light cross-linkable monomers on a front surface of a donor substrate,
b) providing a receiver substrate, and
c) irradiating with a first laser beam of a laser a portion of a back side of the donor substrate to increase the pressure and/or temperature at this portion of the donor substrate to eject a portion of the hydrogel or the hydrogel-forming pre-polymer solution and transfer it to the front side of the receiver substrate; and
d) post-processing the hydrogel or hydrogel-forming pre-polymer on the receiver substrate by irradiating with a second laser beam of a laser at least a portion of the hydrogel-carrying or hydrogel-forming pre-polymer-carrying surface of the receiver substrate to effect at least partial photo-crosslinking of the hydrogel or formation of a hydrogel by at least partially photo-crosslinking the monomers in the pre-polymer solution.

The present invention also resides in an irradiation configuration comprising
a donor substrate coated with a hydrogel or hydrogel-forming pre-polymer solution,
a receiver substrate, having a front surface facing the front surface of the donor substrate, and
a pulsed laser source, which is configured to irradiate with a first laser beam a back side of the donor substrate during a transferring mode of operation and to irradiate a front side of the receiver substrate during a photo-crosslinking operation.

### Detailed Description of the Invention

The present invention relates to a method for printing and photo-crosslinking hydrogels or cell-laden hydrogel constructs on demand using a dual laser beam system. In the inventive method, the first laser beam is used for printing the hydrogel-forming pre-polymer solution or the hydrogel and the second laser beam photo-crosslinks the printed structure.

The hydrogel-forming pre-polymer or the hydrogel is provided on a front surface of a donor substrate. The donor substrate can in principle be made of any material which forms a sufficiently large flat area onto which the hydrogel-forming pre-polymer or the hydrogel is deposited in line with the requirements of the intended purpose and which enables the laser-induced ejection of at least a portion of the hydrogel or hydrogel-forming pre-polymer solution and transfer of same to a receiver substrate.

Materials which are suitable as donor substrate within the context of the present invention are well-known and available to the skilled person. In preferred embodiments of the invention, the donor substrate can include transparent materials which merely or mostly act as a carrier. Within the context of the present invention, the term "transparent" is intended to characterize a material which is permeable for the selected laser wavelength applied within the inventive method. Such completely transparent materials will usually require the presence of a further material, which absorbs energy of the laser beam, i.e. an absorbing layer. Such absorbing layer enables the laser beam to increase the pressure and/or temperature at locations at which such absorbing layer is present and, thus, to eject and transfer the hydrogel-forming pre-polymer or hydrogel from the donor substrate front surface onto the front surface of a receiver substrate. Suitable transparent donor substrate materials include e.g. quartz which is a preferred carrier for use in the inventive laser printing method, or bare glass slides.

The absorbing layer preferably is a thin Au or Ti coating, film or layer on the transparent carrier material, but also other metals can be used, including Ni, Pt, Cu, and Al. As mentioned above, the purpose of the absorbing layer is to absorb energy supplied by the laser beam to induce propulsion of the hydrogel or hydrogel-forming pre-polymer onto the receiver substrate. In addition to metal coatings, films or layers, also non-transparent (again with regard to the selected laser-wavelength) polymeric coatings, films or layers can serve as an absorbing layer, including, but not limited to, polyimide (PI), polyethylene naphthalene (PEN), polyethylene terephthalate (PET).

While preferred donor substrates include a transparent carrier material and an absorbing layer (especially in case of costly Au or Ti absorbing layers), it is also possible to use a donor substrate made of an absorbing material only, e.g. one of the above mentioned polymer materials or a suitable metal substrate or a combination thereof.

Under certain circumstances, it is also possible to only use a transparent carrier as the donor substrate. This option applies in cases in which the material (hydrogel or hydrogel-forming pre-polymer solution) to be transferred from the donor substrate to the receiver substrate itself absorbs the incoming laser wavelength, thus causing an increase in temperature and/or pressure and resulting in propulsion of the material to the receiver substrate.

An even further option within the context of the present invention is the use of a donor substrate which comprises different portions of materials. E.g., the donor material can comprise a transparent carrier material onto which only at certain parts thereof an absorbing layer has been applied. Accordingly, a printing will be limited to the areas comprising the absorbing layer and a respective pattern can be obtained on the receiver substrate.

In the light of the above considerations and the disclosed inventive method, it is well within the skill of the person in the art to choose suitable materials or combinations of materials for the inventive method and the selected laser wavelength.

In the inventive method, the front surface of the receiver substrate is situated opposite to the front surface of the donor substrate. As the laser beam irradiates the backside of the donor substrate, a selected part or pattern of the hydrogel-forming pre-polymer or of the hydrogel is propelled towards the receiver substrate. The propulsion manifests through the formation of an elongated liquid jet or as a solid flyer, depending on the state of the material to be transferred. The donor and the receiver substrates are brought in close proximity or, depending on the application, in contact with each other.

Post-processing is preformed by irradiating at least a portion the hydrogel or the hydrogel-forming pre-polymer on the receiver substrate with a second laser beam of a laser to effect at least partial photo-crosslinking of the hydrogel or the formation of a hydrogel by at least partially photo-crosslinking the monomers in the pre-polymer solution or residual monomers in a hydrogel. It will be apparent to the skilled person that application of one or both of the laser beams allows to provide a desired pattern of hydrogel on the receiver substrate by either only transferring the material onto such part of the receiver substrate, or by only effecting photo-crosslinking at such part and thus only providing a stable material on such parts which are intended to form the desired pattern.

In the present invention, both laser beams can operate at the same wavelength (preferably 532 nm), however, the method can be used with other and different laser wavelengths as well, e.g. depending on a photo-initiator that is employed for photo-crosslinking via the second laser beam. The application of the dual laser beams offers the ability to selectively print and photo-crosslink both hydrogels and cell-laden hydrogels in a rapid manner, while the method can be applied to fabricate constructs that range in size from micrometer up to centimeter.

According to the present invention, a preferred manner of carrying out the inventive method includes removing the donor substrate after the hydrogel or hydrogel-forming pre-polymer solution has been transferred to the receiver substrate. Thus, directing the second laser beam to the receiver substrate to effect photo-crosslinking is facilitated and can be performed using the same laser source. Depending on the configuration of the laser source, however, also other options are applicable and, accordingly, the invention is not limited to this preferred embodiment. For example, a different second laser source which is positioned in a manner which is not affected by the presence of the donor substrate can be used for the photo-crosslinking as will be described in more detail below.

The hydrogels used within the context of the present invention can consist of monomers of natural and/or synthetic (co-)polymers. The same applies with regard to the hydrogel-forming pre-polymer solution which comprises corresponding monomers. The natural and/or synthetic (co-)polymers preferably are able to form 3D network structures, especially network structures which mimic elements of native extracellular matrices and can promote cellular functions.

In the present invention, the hydrogel or hydrogel-forming pre-polymer solution preferably comprises natural (co-)polymers like collagen, Matrigel^{™}, Geltrex^{™}, alginate, hyaluronic acid, chitosan, fibrinogen and fibrin. In further preferred embodiments, the hydrogel or the hydrogel-forming pre-polymer solution comprises synthetic (co-)polymers like PHEMA (poly(hydroxyethylmethacrylate)), PEG (poly(ethylene glycol)), PEGDA (poly(ethylene glycol) diacrylate), VC (vinylcaprolactam), PLMA (platelet lysates modified by addition of methacryloyl groups) and gelatin methacryloyl (GelMA) hydrogels. Furthermore, combinations of natural and synthetic (co-)polymers can be employed within the context of the present invention. The skilled person will be able to choose the desired type and optional combination of hydrogels based on the intended use or application.

To allow for optimal cross-linking of the hydrogel or the hydrogel-forming pre-polymer solution, the inventive method preferably includes one or more photo-initiators. Preferred embodiments include Eosin Y, Irgacure 2959 (I2959) and Lithium phenyl-2,4,6 tri-methylbenzoylphosphinate (LAP). Furthermore, preferably a co-initiator is included. Such co-initiator preferably is selected from the group comprising amine-functionalized co-initiators including but not limited to TEA (triethanolamine). The photo-initiator and, where applicable, co-initiator are usually included in the hydrogel or hydrogel-forming pre-polymer solution which is applied to the front surface of the donor substrate.

In preferred embodiments of the invention, the hydrogel or the hydrogel-forming pre-polymer solution includes cells. Thus, the invention enables laser-bioprinting of cell-laden hydrogels and embedding the cells in an environment, which preserves and enhances cellular functions, like cell adhesion, viability and proliferation, and provides material for a large number of applications, like tissue engineering for transplants.

The cells can be any kind of cell of interest for a certain application. Preferably the cells are selected e.g. from NIH 3T3 fibroblasts, cancer cells (MDA-MB-468, MDA-MB-231, MCF-7, T24). For regenerative purposes, cell types which could be printed include but are not limited to: primary epithelial cells, pancreatic beta-cells, neural cells or neural progenitors, differentiated induced pluripotent stem (iPS) cells, differentiated embryonic stem (ES) cells, primary or differentiated blood-derived mesenchymal stem cells, primary or differentiated adipose tissue-derived mesenchymal stem cells, umbilical cord-derived stem cells, stromal cells including fibroblasts, muscle progenitor or differentiated cells, urothelial cells. For applications such as diagnostics, besides any of the above, cancer cells of any cancer type can be used.

The size of the first laser beam that is used for printing can be between 1 µm and 5 mm in diameter and preferably is 10 µm - 300 µm in diameter, The size of the second laser beam that is employed for the photo-crosslinking can be between 100 µm - 5 mm. Further preferred settings for the laser beams include a fluence of the first laser beam of between 50 to 2500 mJ/cm² and a fluence of the second laser beam of 30 to 5000 mJ/cm². The wavelength of the laser beams can be the same or different and can be selected between 100 and 1600 nm, preferably 266 to 1550 nm, and especially preferably, at least one of the laser beams is set at a wavelength of 532 nm.

In some embodiments, the method of photo-crosslinking includes partial or complete photo-crosslinking of the printed construct using a second laser beam that can be of the same wavelength, or of a different wavelength produced by the same or a different laser source that produces the first laser beam. Thus, the inventive method can be performed using the same or different laser sources for producing the first and the second laser beams. A bioprinter comprising only one laser head can be used for producing both the first and the second laser beam. Alternatively, a bioprinter using two different laser heads can be employed.

Besides the fluence of the laser and the wavelength, the pulse duration is adapted to provide the required printing and/or photo-crosslinking effect. According to the invention, the laser beams application can be from µs to fs pulse durations, and is preferably set to a sub-ns pulse duration at least for the printing via application of the first laser beam. However, depending on the materials to be transferred and especially for the crosslinking, also a plurality of such sub-ns pulses can be applied or a longer duration can be employed as required and advantageous.

In some embodiments, inventive methods of printing can be continuous, where a nonlimiting example can be the printing of a repeating pattern to form a construct of a selected geometry that can be a line, a square, a rectangle or other non-symmetric patterns as well. In another aspect, methods of printing include the deposition of the hydrogel or cell-laden hydrogel in the form of a matrix comprising an array of printed droplets.

In a further preferred embodiment of the invention, more than one layer of hydrogel can be printed or formed on the receiver substrate either at different locations of the receiver substrate, or on top of each other. In such context, e.g. one layer can include cells, whereas another layer forms some sort of protective top layer. Also, first forming a basic layer on the receiver substrate onto which a cell-laden hydrogel layer is deposited, optionally followed by another hydrogel layer without cells can be considered and also depositing different layers including different cells, optionally including "pure" hydrogel layers on the top, the bottom and/or in between cell-including layers can be contemplated within the context of the present invention. Forming different arrays of different cell types at different locations of the receiver substrate is another option which can also be combined with base or top layers of "pure" hydrogel. In addition to hydrogels with or without cells, also different hydrogel polymer compositions can be used and further substances can be included in the "pure" hydrogels as well as in the cell-laden hydrogels.

It is also possible to interconnect such layers via photo-crosslinking of either the same or different hydrogels or hydrogel-forming pre-polymer solutions.

In a further embodiment, a receiver substrate can be used which includes a modification which allows for crosslinking the substrate itself with the hydrogel materials and, thus, to provide for an especially firm fixation of the hydrogel on the receiver substrate. The inventive method allows to adapt the materials and designs within the laser bio-printing method as required for the intended application.

In an especially preferred embodiment of the invention, gelatin methacryloyl with vinylcaprolactam (VC) as a hydrogel co-monomers, Eosin Y as the photoinitiator and triethanolamine (TEA) as a co-initiator are used and the photocrosslinking is effected via free radical chain reaction polymerization, upon excitation of the photoinitiator with visible light irradiation. This preferred embodiment shall be understood as providing an especially advantageous combination of reactants and conditions, but also as disclosing a list of advantageous and preferred individual reactants and conditions for combination with other specific embodiments mentioned elsewhere in this description of the invention. The cell line employed in the examples described below were NIH 3T3 fibroblasts, however other cell lines may be used as well (cancer cell line T24). The present invention can be used with other hydrogels and/or combinations of hydrogels and photoinitiators and is not limited to the above-mentioned materials and cell lines.

A further aspect of the invention relates to an irradiation configuration comprising:
a donor substrate as defined above
a receiver substrate, having a front surface facing the front surface of the donor substrate, a pulsed laser source, configured to irradiate with a first laser beam a back side of the donor substrate during a transferring mode of operation and to irradiate a front side of the receiver substrate during a photo-crosslinking operation.

The donor substrate, the hydrogel or hydrogel-forming pre-polymer solution, the receiver substrate as well as the settings of a laser source to produce the laser beams have been discussed above in detail with reference to the inventive method. All such disclosure applies equally within the context of the irradiation configuration.

Furthermore, in one additional aspect, disclosed herein is a bioprinter comprising at least one laser head, wherein a computer-controlled mirror is used for optical path switching between the printing and the photopolymerization laser heads. Another aspect, disclosed herein is a bioprinter comprising at least one laser head, wherein a computer-controlled beam-splitting optical component is used to switch the laser wavelength between the printing and the photopolymerization laser heads.

In addition, disclosed herein is a bioprinter comprising two different laser heads, wherein a computer-controlled mirror is used to switch between the two laser wavelengths, from which, one is employed for the printing and the second for the photopolymerization process. The optical path for the propagation of both laser beams in the present invention involves one or more optionally employed optical attenuation plates for adjusting the laser intensity.

In yet another aspect, the invention provides a method for fabricating a hydrogel construct comprising steps of: coating the pre-polymer solution on a carrier substrate and transferring it onto a receiving substrate by means of a first laser head, wherein the pre-polymer solution comprises a visible light cross-linkable material that is exposed to visible light irradiation, after printing, with visible light irradiation produced by a second laser beam that can be of the same wavelength, or of a different wavelength produced by the same or a different laser source to initiate the partial or complete photo-crosslinking of the previously printed hydrogel construct. Also in regard of this aspect, the explanations and definitions provided above for other aspects of the invention apply with equal measure.

In certain embodiments, the hydrogel pre-polymer comprises a visible light cross-linkable material and cells. In further aspects, the method further comprises steps of printing the cell-laden hydrogel solution using a first laser head from a carrier substrate to a receiving substrate and subsequently exposing the printed cell-laden hydrogel solution to visible light irradiation by means of a second laser beam that can be of the same wavelength, or of a different wavelength produced by the same or a different laser source to initiate the partial or complete photo-crosslinking of the previously printed hydrogel construct.

In another aspect disclosed herein, cells are printed by a laser head on top of a previously printed and photo-crosslinked hydrogel, constructed using a method that comprises steps of: coating the pre-polymer solution on a carrier substrate and transferring it onto a receiving substrate by means of a first laser head, wherein the pre-polymer solution comprises a visible light cross-linkable material that is exposed to visible light irradiation, after printing, produced by a second laser beam that can be of the same wavelength, or of a different wavelength produced by the same or a different laser source to initiate the partial or complete photo-crosslinking of the previously printed hydrogel construct.

In summary, the present invention discloses the following items:
Item 1. Method of forming a hydrogel on a receiver substrate, the method comprising:
   a) providing a hydrogel or a hydrogel-forming pre-polymer solution comprising visible light cross-linkable monomers on a front surface of a donor substrate, the donor substrate comprising an energy absorbing layer or portion and/or a transparent carrier portion,
   b) providing a receiver substrate ,
   c) irradiating with a first laser beam of a laser a portion of a back side of the transparent portion of the donor substrate to increase the pressure and/or temperature at this portion of the donor substrate to eject a portion of the hydrogel or the hydrogel-forming pre-polymer solution and transfer it to the front side of the receiver substrate; and
   d) post-processing the hydrogel or hydrogel-forming pre-polymer on the receiver substrate by irradiating with a second laser beam of a laser at least a portion of the hydrogel or hydrogel-forming pre-polymer-carrying surface of the receiver substrate to effect at least partial photo-crosslinking of the hydrogel or formation of a hydrogel by at least partially photo-crosslinking the monomers in the pre-polymer solution.
Item 2. Method according to item 1, further comprising removing the donor substrate after the portion of the hydrogel or hydrogel-forming pre-polymer solution has been transferred to the receiver substrate.
Item 3. Method according to items 1 or 2, the hydrogel consists of or the hydrogel-forming pre-polymer solution comprises monomers of natural and/or synthetic (co)polymers.
Item 4. Method according to item 3, wherein the natural and/or synthetic (co-)polymers form 3D network structures, preferably network structures which mimic elements of native extracellular matrices and can promote cellular functions.
Item 5. Method according to any one of items 3 or 4, wherein natural (co-)polymers comprise collagen, Matrigel^{™}, Geltrex^{™}, alginate, hyaluronic acid, chitosan, fibrinogen and fibrin, and/or synthetic (co-)polymers comprise PHEMA (poly(hydroxyethylmethacrylate)), PEG (poly(ethylene glycol)), PEGDA (poly(ethylene glycol) diacrylate), VC (vinylcaprolactam), PLMA (platelet lysates modified by addition of methacryloyl groups) and gelatin methacryloyl (GelMA) hydrogels.
Item 6. Method according to any one of items 1 to 5, wherein the method includes providing a photoinitiator and, optionally, a co-initiator.
Item 7. Method according to item 6, wherein the photoinitiator is selected from the group comprising Eosin Y, Irgacure 2959 (I2959) and Lithium phenyl-2,4,6 tri-methylbenzoylphosphinate (LAP), and the co-initiator is selected from the group comprising amine-functionalized *co-initiators including but not limited to* TEA (triethanolamine).
Item 8. Method according to any one of the preceding items, wherein the hydrogel or the hydrogel-forming pre-polymer comprises cells.
Item 9. Method according to item 8, wherein the cells are selected from NIH 3T3 fibroblasts, cancer cells (MDA-MB-468, MDA-MB-231, MCF-7, T24), human embryonic stem cells, human mesenchymal stem cells, adipose tissue-derived mesenchymal *stem cells, human umbilical vein endothelial cells.*
Item 10. Method according to any one of the preceding items, wherein the size of the first laser beam is between 1 µm to 5 mm in diameter, preferably 10 to 300 µm in diameter and/or the size of the second laser beam is between 100 µm and 5 mm in diameter.
Item 11. Method according to any one of the preceding items, comprising setting the fluence of the first laser beam to 50 to 1500 mJ/cm².
Item 12. Method according to any one of the preceding items, comprising setting the fluence of the second laser beam to 30 to 500 mJ/cm².
Item 13. Method according to any one of the preceding claims, comprising selecting the first and second laser beams at the same or different wavelengths between 266 and 1600 nm nm especially both at 532 nm.
Item 14. Method according to any one of the preceding items, wherein the first and second laser beams are produced by the same or different laser sources.
Item 15. Method according to any one of the preceding items, comprising applying each of the laser beams at sub-ns pulse duration.
Item 16. Method according to any one of the preceding items, which is performed continuously to form a construct of a selected geometry like a line, a square, a rectangle of a non-symmetric continuous pattern, or discontinuously to form a matrix or array of printed droplets or areas on the receiver substrate.
Item 17. Method according to any one of the preceding items, comprising forming more than one layer of at least partially photo-crosslinked hydrogel on the receiver substrate.
Item 18. Method according to item 17, wherein the more than one layer of hydrogel are formed either on top of each other or at a different location of the receiver substrate, or a combination of both.
Item 19. Method according to items 17 or 18, wherein the more than one layers of hydrogel differ in at least one of their hydrogel-forming polymer composition, presence or absence of cells contained in the hydrogels, cell type and presence or absence of further substances.
Item 20. An irradiation configuration comprising:
   a donor substrate, having a transparent portion coated with a hydrogel or hydrogel-forming pre-polymer solution,
   a receiver substrate, having a front surface facing the front surface of the donor substrate,
   a pulsed laser source, configured to irradiate with a first laser beam a back side of the donor substrate during a transferring mode of operation and to irradiate a front side of the receiver substrate during a photo-crosslinking operation.
Item 21. An irradiation configuration according to item 20, further comprising at least one type of cells included with at least part of the hydrogel or hydrogel-forming pre-polymer solution.
Item 22. An irradiation configuration according to items 20 or 21, comprising a first stage to hold the donor substrate and a second stage to hold the receiver substrate, wherein the stages are independently and relatively moveable with respect to the laser source.

The following examples further illustrate the invention, however, are not intended to limit the scope thereof.

### Examples

### Example 1 - Laser printing and laser photocrosslinking of gelatin methacryloyl hydrogels

An example of the present invention includes the laser transfer of a gelatin methacryloyl-based hydrogels and its subsequent photo-crosslinking through a polymerization process. This hydrogel is a biocompatible surface onto which cells can adhere and proliferate and can be used in transplants for tissue engineering.

Initially gelatin-methacryloyl (10% w/v) was dissolved in the photo-initiator solution that contained 1.5% w/v triethanolamine, 1% w/v N-vinylcaprolactam and 0.1 mM Eosin Y disodium salt in Dulbecco's phosphate-buffered saline (DPBS). The pre-polymer solution was then placed in a water bath at 37 °C and subsequently 10 µl of the solution were drop-casted on the donor substrate (circular quartz substrate, 25.4 mm diameter, 1 mm thickness) to produce a uniform thin film.

The apparatus employed for the transfer of the pre-polymer solution on the receiver substrate included a pulsed laser source operating at 532 nm and sub-ns pulse duration. This laser wavelength was selected since a thin Au coating was applied on the circular quartz substrate as an absorbing layer to absorb the laser energy and induce the propulsion of the material towards the receiver substrate. The laser beam was focused using an objective lens on the donor substrate and the laser spot size could be varied between 1-300 µm. The system operated at mask projection conditions and the process could be viewed in real time using a high-power imaging system based on the inverted microscope principle with the aid of a CCD camera. The energy density of the laser may be adjusted using a rotating waveplate and a polarizer between 350-2500 mJ/cm².

For the photo-crosslinking, after the transfer of the pre-polymer solution on selected areas of the receiver substrate the donor substrate is removed and a second laser beam is used to crosslink the transferred pre-polymer solution using the 532 nm (sub-ns pulse duration) wavelength on the areas of the receiver substrate where the pre-polymer was previously transferred.

The laser spot on the receiver substrate can be varied between 100 µm - 5 mm and the laser fluence may be varied between 30-5000 mJ/cm². The process was repeated multiple times to crosslink the whole area of the receiver substrate where the pre-polymer has been previously transferred onto. The process was monitored using the high-power imaging system described above. The photo-crosslinked structure was finally washed in DPBS for 10 min.

### Example 2 - Laser printing and laser photocrosslinking of gelatin methacryloyl hydrogels and subsequent laser printing of NIH 3T3 fibroblasts

An example of the present invention includes the laser transfer of a gelatin methacryloyl-based hydrogels and its subsequent photo-crosslinking through a polymerization process followed by laser printing of NIH 3T3 fibroblasts on top of the laser printed and laser photocrosslinked hydrogel.

Initially gelatin-methacryloyl (10% w/v) was dissolved in the photo-initiator solution that contained 1.5% w/v triethanolamine, 1% w/v N-vinylcaprolactam and 0.1 mM Eosin Y disodium salt in Dulbecco's phosphate-buffered saline (DPBS). The pre-polymer solution was then placed in a water bath at 37 °C and subsequently 10 µl of the solution were drop-casted on the donor substrate (circular quartz substrate, 25.4 mm diameter, 1 mm thickness) to produce a uniform thin film.

The apparatus employed for the transfer of the pre-polymer solution on the receiver substrate included a pulsed laser source operating at 532 nm and sub-ns pulse duration. This laser wavelength was selected since a thin Au coating was applied on the circular quartz substrate as an absorbing layer to absorb the laser energy and induce the propulsion of the material towards the receiver substrate. The laser beam was focused using an objective lens on the donor substrate and the laser spot size could be varied between 1-300 µm. The system operated at mask projection conditions and the process could be viewed in real time using a high-power imaging system based on the inverted microscope principle with the aid of a CCD camera. The energy density of the laser may be adjusted using a rotating waveplate and a polarizer between 350-2500 mJ/cm².

For the photo-crosslinking, after the transfer of the pre-polymer solution on selected areas of the receiver substrate the donor substrate is removed and a second laser beam is used to crosslink the transferred pre-polymer solution using the 532 nm (sub-ns pulse duration) wavelength on the areas of the receiver substrate where the pre-polymer was previously transferred.

The laser spot on the receiver substrate can be varied between 100 µm - 5 mm and the laser fluence may be varied between 30-5000 mJ/cm2. The process was repeated multiple times to crosslink the whole area of the receiver substrate where the pre-polymer has been previously transferred onto. The process was monitored using the high-power imaging system described above. The photo-crosslinked structure was finally washed in DPBS for 10 min.

After the photo-crosslinking step, 10 µl of NIH 3T3 fibroblasts cell solution (2 x 10⁶ cells/ml) were laser printed on top of the previously printed and photocrosslinked hydrogel. After an 1 h incubation, cell culture media (300 µl) was added to the sample and the sample was kept for 7 days at 37 °C and 5% CO2. Cell proliferation (Presto Blue assay), viability (live/dead assay) and adhesion of the cell seeded hydrogel were subsequently tested and a cell viability of >95% was observed after 1, 5 and 8 days.

### Example 3 - Laser printing and laser photocrosslinking of cell-laden gelatin methacryloyl hydrogels

Another example of the present invention includes the laser transfer of a cell-laden gelatin methacryloyl-based hydrogel and its subsequent photo-crosslinking through a polymerization process to obtain a cross-linked cell-laden hydrogel construct.

Initially NIH 3T3 fibroblasts were suspended in 10% w/v gelatin-methacryloyl pre-polymer that contained the photo-initiator solution described in example 1 at a concentration of 2 x 106 cells/ml. The pre-polymer solution was then placed in a water bath at 37 °C and subsequently 10 µl of the solution were drop-casted on the donor substrate (circular quartz substrate, 25.4 mm diameter, 1 mm thickness) to produce a uniform thin film.

The apparatus employed for the transfer of the cell-laden precursor solution on the receiver substrate included a pulsed laser source operating at 532 nm and sub-ns pulse duration. The laser beam was focused using an objective lens on the donor substrate and the laser spot size could be varied between 1-300 µm. The system operated at mask projection conditions and the process could be viewed in real time using a high-power imaging system based on the inverted microscope principle with the aid of a CCD camera. The energy density of the laser may be adjusted using a rotating waveplate and a polarizer between 350-2500 mJ/cm2.

For the photo-crosslinking, after the transfer of the cell-laden precursor solution on selected areas of the receiver substrate the donor substrate is removed and a second laser beam is used to crosslink the transferred pre-polymer solution using the 532 nm (sub-ns pulse duration) wavelength on the areas of the receiver substrate where the pre-polymer was previously transferred.

The laser spot on the receiver substrate can be varied between 100 µm - 5 mm and the laser fluence may be varied between 30-5000 mJ/cm2. The process was repeated multiple times to crosslink the whole area of the receiver substrate where the cell-laden precursor solution has been previously transferred onto. The process was monitored using the high-power imaging system described above. The photocrosslinked cell-laden hydrogel was washed four times with DPBS.

After the photo-crosslinking step, cell viability was tested using a live/dead assay kit and a cell viability >95% was observed after 1, 5 and 8 days.

## Claims

1. Method of forming a hydrogel on a receiver substrate, the method comprising:
a) providing a hydrogel or a hydrogel-forming pre-polymer solution comprising visible light cross-linkable monomers on a front surface of a donor substrate
b) providing a receiver substrate ,
c) irradiating with a first laser beam of a laser a portion of a back side of the donor substrate to increase the pressure and/or temperature at this portion of the donor substrate to eject a portion of the hydrogel or the hydrogel-forming pre-polymer solution and transfer it to the front side of the receiver substrate; and
d) post-processing the hydrogel or hydrogel-forming pre-polymer on the receiver substrate by irradiating with a second laser beam of a laser at least a portion of the hydrogel or hydrogel-forming pre-polymer-carrying surface of the receiver substrate to effect at least partial photo-crosslinking of the hydrogel or formation of a hydrogel by at least partially photo-crosslinking the monomers in the pre-polymer solution.

2. Method according to claim 1 , the hydrogel consists of or the hydrogel-forming pre-polymer solution comprises monomers of natural and/or synthetic (co-)polymers, wherein preferably the natural and/or synthetic (co-)polymers form 3D network structures, more preferably network structures which mimic elements of native extracellular matrices and can promote cellular functions.

3. Method according to any one of claims 2, wherein natural (co-)polymers comprise collagen, Matrigel^{™}, Geltrex^{™}, alginate, hyaluronic acid, chitosan, fibrinogen and fibrin, and/or synthetic (co-)polymers comprise PHEMA (poly(hydroxyethylmethacrylate)), PEG (poly(ethylene glycol)), PEGDA (poly(ethylene glycol) diacrylate), VC (vinylcaprolactam), PLMA (platelet lysates modified by addition of methacryloyl groups) and gelatin methacryloyl (GelMA) hydrogels.

4. Method according to any one of claims 1 to 3, wherein the method includes providing a photoinitiator and, optionally, a co-initiator, wherein preferably the photoinitiator is selected from the group comprising Eosin Y, Irgacure 2959 (I2959) and Lithium phenyl-2,4,6 tri-methylbenzoylphosphinate (LAP), and wherein preferably the co-initiator is selected from the group comprising amine-functionalized *co-initiators including but not limited to* TEA (triethanolamine).

5. Method according to any one of the preceding claims, wherein the hydrogel or the hydrogel-forming pre-polymer comprises cells.

6. Method according to claim 5, wherein, for regenerative purposes, cell types include primary epithelial cells, pancreatic beta-cells, neural cells or neural progenitors, differentiated induced pluripotent stem (iPS) cells, differentiated embryonic stem (ES) cells, primary or differentiated blood-derived mesenchymal stem cells, primary or differentiated adipose tissue-derived mesenchymal stem cells, umbilical cord-derived stem cells, stromal cells including fibroblasts, muscle progenitor or differentiated cells, urothelial cells, and, for applications such as diagnostics, besides any of the above, cancer cells of any cancer type.

7. Method according to any one of the preceding claims, wherein the size of the first laser beam is between 1 µm to 5 mm in diameter, preferably 10 to 300 µm in diameter and/or the size of the second laser beam is between 100 µm and 5 mm in diameter.

8. Method according to any one of the preceding claims, comprising setting the fluence of the first laser beam to 50 to 1500 mJ/cm² and/or setting the fluence of the second laser beam to 30 to 500 mJ/cm².

9. Method according to any one of the preceding claims, comprising selecting the first and second laser beams at the same or different wavelengths between 266 nm and 1600 nm, especially both at 532 nm, and/or wherein the first and second laser beams are produced by the same or different laser sources.

10. Method according to any one of the preceding claims, comprising applying each of the laser beams at sub-ns pulse duration.

11. Method according to any one of the preceding claims, which is performed continuously to form a construct of a selected geometry like a line, a square, a rectangle of a non-symmetric continuous pattern, or discontinuously to form a matrix or array of printed droplets or areas on the receiver substrate.

12. Method according to any one of the preceding claims, comprising forming more than one layer of at least partially photo-crosslinked hydrogel on the receiver substrate, wherein the more than one layer of hydrogel are formed either on top of each other or at a different location of the receiver substrate, or a combination of both.

13. Method according to claim 12, wherein the more than one layers of hydrogel differ in at least one of their hydrogel-forming polymer composition, presence or absence of cells contained in the hydrogels, cell type and presence or absence of further substances.

14. An irradiation configuration comprising:
a donor substrate coated with a hydrogel or hydrogel-forming pre-polymer solution,
a receiver substrate, having a front surface facing the front surface of the donor substrate,
a pulsed laser source, configured to irradiate with a first laser beam a back side of the donor substrate during a transferring mode of operation and to irradiate a front side of the receiver substrate during a photo-crosslinking operation.

15. An irradiation configuration according to claim 14, further comprising at least one type of cells included with at least part of the hydrogel or hydrogel-forming pre-polymer solution and/or comprising a first stage to hold the donor substrate and a second stage to hold the receiver substrate, wherein the stages are independently and relatively moveable with respect to the laser source.
